# FASCICULE DE BREVET EUROPEEN

(11) **EP 0 707 466 B1**
(45) Date de publication et mention de la délivrance du brevet: **04.02.1998**
(21) Numéro de dépôt: 94922894.4
(22) Date de dépôt: 08.07.1994
(51) Int. Cl.: A61F 13/15

(54) **ARTICLE D'HYGIENE ABSORBANT JETABLE**
WEGWERFBARER ABSORBIERENDER SANITÄRARTIKEL
DISPOSABLE ABSORBENT SANITARY ARTICLE

(30) Priorité: 09.07.1993 FR 9308723
(43) Date de publication de la demande: 24.04.1996
(73) Titulaire: SCA Mölnlycke, 59497 Linselles Cédex (FR)
(72) Inventeur: MARTIN, Françoise, F-59110 La Madeleine (FR)
(74) Mandataire: Casalonga, Axel
(86) Numéro de dépôt international: FR9400856
(87) Numéro de publication internationale: WO9501768

(56) Documents cités:
- EP-A- 0 190 881
- EP-A- 0 371 871
- EP-A- 0 391 476
- GB-A- 2 161 059
- US-A- 4 662 877
- US-A- 4 846 825

## Description

La présente invention concerne un article d'hygiène absorbant jetable, notamment une couche-culotte jetable pour enfant en bas âge ou pour incontinent adulte, qui comporte un coussin absorbant disposé sur une feuille extérieure de support, imperméable aux liquides. Elle concerne plus particulièrement un article d'hygiène perfectionné permettant d'assurer une très bonne étanchéité au niveau des cuisses de l'utilisateur, et donc d'éviter tout risque de fuite d'urine et de matières fécales le long des jambes de celui-ci.

On a déjà proposé de multiples dispositions visant à réaliser cette étanchéité. La première a consisté dans la mise en place d'élastiques d'entrejambes destinés à réaliser une certaine contention de l'article autour des jambes de l'utilisateur. L'article d'hygiène comporte dans le sens longitudinal une partie centrale qui correspond à la zone d'entrejambes et deux parties extrêmes, l'une avant et l'autre arrière, qui correspondent aux zones de positionnement et de fixation autour de la taille de l'usager. Les éléments élastiques en question sont fixés longitudinalement le long des deux bords extérieurs de la feuille support au moins dans la partie centrale.

Cependant cette première disposition s'est avérée insuffisante dans la mesure où la force de contention susceptible d'être exercée sur la jambe de l'utilisateur était nécessairement limitée et que d'autre part il se produisait des plis générateurs de fuites.

Comme autre disposition, on a déjà proposé de réaliser, en complément des élastiques d'entrejambes, des rabats longitudinaux destinés à former une barrière empêchant la diffusion transversale des matières fécales. De tels rabats sont par exemple décrits dans le document GB 2 161 059, dans lequel, de préférence, chaque rabat est constitué en repliant une feuille de protection, perméable aux liquides, qui s'étend par dessus le coussin absorbant et qui a la même forme extérieure que la feuille support perméable aux liquides. L'effet de barrière est obtenu notamment en interposant à l'intérieur du pli, formant rabat, un élément élastique qui a pour effet de relever le rabat lorsque l'article d'hygiène est positionné sur l'utilisateur.

Comme autre disposition, décrite dans le document US 4 662 877, on a disposé au-dessus du coussin absorbant et de la feuille support imperméable une feuille de couverture hydrophobe qui est fixée à la feuille support de manière à former une structure sandwich. La feuille de couverture a une ouverture dans la zone centrale d'entrejambes et comporte, fixés le long des bords longitudinaux de cette ouverture, des éléments élastiques qui sont disposés à l'état tendu de telle sorte qu'ils ont tendance à relever lesdits bords longitudinaux lorsque l'article est placé sur l'utilisateur. Ainsi on a également un effet de barrière, cet effet étant encore amélioré par rapport au document GB 2 161 059 dans la mesure où l'on a non seulement des rabats longitudinaux mais également une certaine protection transversale du fait de la présence de la feuille de couverture sur les portions extrêmes du coussin absorbant.

Cependant même s'il présente une structure particulièrement favorable, l'article d'hygiène selon le document US 4 662 877 ne permet pas d'obtenir une parfaite étanchéité, comme cela est souhaité par le demandeur. En effet, même si les matières fécales sont concentrées dans la poche intérieure donnant sur l'ouverture de la feuille de couverture, il n'en reste pas moins vrai que lorsque l'utilisateur séjourne en position assise il se produit des migrations de liquide par delà la feuille de couverture et des élastiques d'entrejambes.

Le but que s'est fixé le demandeur est de proposer un article d'hygiène qui pallie l'inconvénient précité. Il s'agit de manière connue par le document US 4 662 877, d'un article hygiène absorbant jetable, notamment une couche-culotte, qui comporte un coussin absorbant disposé entre une feuille extérieure support imperméable aux liquides et une feuille de couverture hydrophobe, les deux dites feuilles ayant la même forme et délimitant dans le sens longitudinal une partie centrale qui correspond à la zone d'entrejambes et deux parties extrêmes, l'une avant et l'autre arrière, qui correspondent aux zones de positionnement et de fixation autour de la taille de l'usager. La feuille support et la feuille de couverture sont solidarisées au moins le long de leur pourtour et la feuille de couverture comporte une ouverture centrale qui s'étend au-dessus du coussin absorbant; de plus l'article d'hygiène comporte des jeux de moyens élastiques, disposés symétriquement par rapport à l'axe longitudinal de l'article : le premier jeu dit jeu d'entrejambes est fixé à la feuille support et à la feuille de couverture le long de leurs bords extérieurs au moins dans la partie centrale et le deuxième jeu est fixé à la feuille de couverture le long des bords longitudinaux de l'ouverture centrale.

De manière caractéristique l'article d'hygiène selon l'invention comporte un jeu de deux rabats hydrophobes, symétriques par rapport à l'axe longitudinal de l'article, chaque rabat étant constitué d'une bande d'un matériau hydrophobe et éventuellement imperméable aux liquides, dont les deux bords longitudinaux sont fixés pour l'un à proximité du bord longitudinal extérieur du coussin absorbant, au moins dans la zone d'entrejambes, et pour l'autre sur la face inférieure de la feuille de couverture à proximité des moyens élastiques du second jeu. Ainsi on obtient un double effet de barrière dont la mise en oeuvre est obtenue à l'aide du seul deuxième jeu de moyens élastiques. En effet celui-ci permet de relever non seulement les deux montants latéraux de la feuille de couverture, situés entre l'ouverture centrale et le premier jeu de moyens élastiques, mais encore les deux rabats hydrophobes. On obtient donc, comme dans le document US 4 662 877 une poche intérieure donnant sur l'ouverture de la feuille de couverture, mais cette poche est délimitée intérieurement par les deux rabats hydrophobes et extérieurement par les deux montants latéraux de la feuille de couverture. C'est la présence de cette double barrière qui permet d'obtenir l'étanchéité améliorée visée par le demandeur.

Le coussin absorbant est généralement recouvert d'une feuille de protection perméable aux liquides. Selon l'invention, de préférence, cette feuille de protection a une largeur qui est supérieure à celle du coussin de manière à délimiter une bande centrale, perméable aux liquides, et deux bandes latérales qui, elles, ne sont pas perméables aux liquides mais hydrophobes et éventuellement imperméables aux liquides; de plus ladite bande centrale est fixée longitudinalement à proximité des deux bords extérieurs longitudinaux du coussin absorbant en sorte que les deux bandes latérales précitées constituent le jeu de deux rabats hydrophobes caractéristiques de l'invention. Cette disposition particulière simplifie la fabrication de l'article selon l'invention puisque la réalisation des deux rabats longitudinaux ne nécessite pas, dans ce cas, de constituants supplémentaires.

De préférence chaque bande latérale de la feuille de protection comporte un pli longitudinal; la pliure correspondante est fixée longitudinalement sur la feuille support imperméable le long du coussin absorbant extérieurement à celui-ci, au moins dans la zone d'entrejambes; les deux flancs du pli longitudinal sont fixés l'un à l'autre au niveau et sur toute la longueur de la ligne de fixation de la feuille de protection sur le coussin absorbant.

Cette version particulière vise à améliorer l'étanchéité aux liquides de l'article d'hygiène et donc l'élimination de tous risques éventuels de fuite d'urine, qui pourraient se produire au niveau du coussin absorbant le long de la feuille support imperméable. Ceci est obtenu grâce au pli longitudinal, en double épaisseur, formant une double barrière contre les migrations possibles de liquide provenant du coussin absorbant.

Avantageusement un troisième jeu de moyens élastiques est fixé longitudinalement à la feuille de couverture entre la ligne de fixation du rabat à ladite feuille de couverture et le premier jeu de moyens élastiques. La mise en oeuvre de ce troisième jeu de moyens élastiques permet de constituer latéralement, le long du coussin absorbant, deux tunnels aptes à constituer des réservoirs tampons. Dans le cas où les rabats hydrophobes sont dans un matériaux perméable aux gaz, ils constituent des tunnels d'aération permettant d'obtenir un échange gazeux entre l'intérieur et l'extérieur de l'article d'hygiène et donc l'évacuation progressive de l'humidité régnant dans la poche intérieure.
D'autres modes particuliers de réalisation selon l'invention sont indiqués dans les revendications 4, 6 à 8.

La présente invention sera mieux comprise à la lecture de la description qui va être faite d'exemples de réalisation d'une couche-culotte à feuille de couverture et à feuille de protection formant rabat, illustrés par les dessins annexés dans lesquels :
- la figure 1 est une représentation d'une réalisation de la couche-culotte à l'état tendu selon une vue en plan,
- la figure 2 est une demi-vue en coupe selon la ligne II-II de la figure 1,
- la figure 3 représente la couche-culotte selon la figure 2 à l'état non tendu,
- la figure 4 est une demi-vue en coupe, analogue à celle de la figure 2, d'une autre réalisation de l'invention, et
- la figure 5 est une demi-vue en coupe, analogue à celle de la figure 2, d'encore une autre réalisation de l'invention.

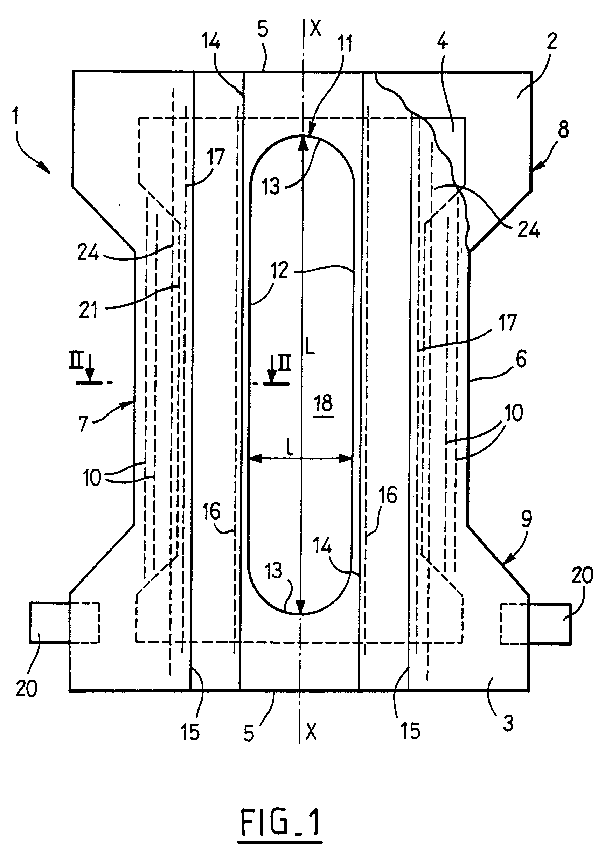

La couche-culotte 1 qui est illustrée à la figure 1 se compose de manière connue par le document US 4 662 877 d'une feuille de support extérieure 2 imperméable aux liquides, par exemple un film de polyéthylène, une feuille de couverture intérieure 3, perméable aux liquides, par exemple une feuille de non-tissé hydrophobe et un coussin absorbant 4 disposé entre les deux feuilles précitées 2 et 3.

La feuille de support extérieure 2 et la feuille de couverture intérieure 3 ont les mêmes dimensions et la même forme générale, étant sensiblement rectangulaires avec deux bords transversaux rectilignes opposés 5 et deux bords longitudinaux opposés comportant chacun une échancrure 6 définissant une zone d'entrejambes 7 de largeur réduite et des parties avant 8 et arrière 9 qui correspondent aux zones de positionnement et de fixation de la couche-culotte 1 autour de la taille de l'usager, à l'aide d'attaches adhésives 20.

Le coussin absorbant 4 est composé par exemple de pâte fluff de cellulose, contenant éventuellement un polymère superabsorbant. Dans l'exemple illustré, il a une forme en sablier, similaire à celle des feuilles de support 2 et de couverture 3. De plus il est collé à la feuille de support 2, symétriquement par rapport à l'axe XX de symétrie de la couche-culotte 1.

La couche-culotte 1 comporte également des élastiques d'entrejambes 10, qui sont fixés entre la feuille support 2 et la feuille de couverture intérieure 3 longitudinalement le long de chacun des bords extérieurs de la couche-culotte 1 dans la zone d'entrejambes 7. La feuille de couverture intérieure 3 est pourvue d'une ouverture centrale 11, obtenue par exemple par découpe. Cette ouverture centrale 11 a une forme oblongue avec deux bords longitudinaux 12, rectilignes, et deux bords semi-circulaires 13. Ladite ouverture centrale 11 s'étend, symétriquement par rapport à l'axe XX, dans le sens longitudinal de la couche-culotte 1, par dessus le coussin absorbant 4, sur une longueur L qui est supérieure à la zone d'entrejambes 7. La largeur 1 de l'ouverture centrale 11, entre les deux bords longitudinaux 12, est par exemple de l'ordre de la moitié de la largeur du coussin absorbant dans la zone d'entrejambes 7.

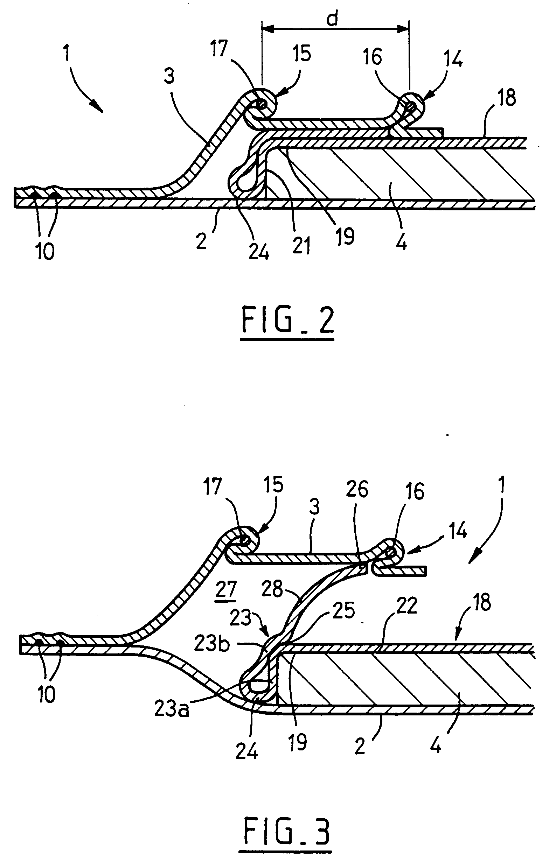

Comme cela apparaît explicitement à l'examen des figures 2 et 3, la feuille de couverture intérieure 3 comporte de part et d'autre de l'ouverture centrale 11 deux jeux de deux plis longitudinaux 14, 15 en forme de Z, symétriques par rapport à l'axe XX, dans lesquels plis longitudinaux sont fixés, par exemple par collage, des éléments élastiques 16, 17. Ces éléments élastiques 16, 17 sont fixés à l'état tendu et solidarisés sur toute leur longueur dans les plis respectivement 14, 15 de ladite feuille de couverture 3. La fixation des éléments élastiques 16, 17 sur la feuille de couverture intérieure 3 dans des plis en forme de Z n'est cependant pas limitative. Tout autre type de fixation serait envisageable, notamment dans des plis en forme de S ou par fixation directe sans pli.

La disposition respective des plis 14 et 15 est la suivante. Le premier pli 14 et l'élément élastique correspondant 16 sont disposés à proximité immédiate d'un bord longitudinal 12 de l'ouverture centrale 11. Le second pli 15 et l'élément élastique correspondant 17 sont éloignés d'une distance déterminée d du premier pli 14 et de son élément élastique 16. Dans l'exemple illustré cette distance d est sensiblement égale à la distance séparant les éléments élastiques d'entrejambes 10 et le second élément élastique 17.

De plus le second élément élastique 17 est fixé, à l'intérieur du pli 15 à l'état tendu avec un allongement qui est de l'ordre de 20% supérieur à l'allongement que présente le premier élément élastique 16 lorsqu'il est fixé à l'état tendu à l'intérieur du premier pli 14. Comparativement les éléments élastiques 10 d'entrejambes présentent lorsqu'ils sont fixés à l'état tendu, un allongement qui est lui-même supérieur à celui du second élément élastique 17.

La couche-culotte 1 de l'invention comporte également une feuille de protection 18, consistant par exemple en un non-tissé de forme rectangulaire.

Cette feuille de protection 18 se trouve placée entre la feuille de couverture intérieure 3 et le coussin absorbant 4; plus pécisément elle recouvre le coussin absorbant 4, auquel elle est fixée selon des lignes de fixation longitudinales, parmi lesquelles au moins une ligne de fixation 19 qui est située sur le dessus du coussin absorbant 4 le long de son bord extérieur 21. La feuille de protection 18 a une largeur nettement supérieure à celle du coussin absorbant. De plus alors que la bande centrale 22 de cette feuille de protection 18 qui se trouve à l'aplomb du coussin absorbant 4 est hydrophile, les deux bandes latérales 23 qui s'étendent au-delà de cette bande centrale 22, de part et d'autre des lignes de fixation 19 sont hydrophobes. Le caractère hydrophobe voire même l'imperméabilité aux liquides peut notamment être obtenu par un traitement préalable de la feuille de protection 18. Chacune des bandes latérales 23 est repliée sur elle-même, selon une ligne de pliure longitudinale 24 qui est fixée à la feuille support 2 le long du coussin absorbant 4 extérieurement à celui-ci, au moins dans la zone d'entrejambes 7. De plus les deux flancs 23a et 23b de la bande latérale 23 sont fixés l'un à l'autre selon une ligne de fixation 25 qui est située au niveau et qui s'étend sur toute la longueur de la ligne de fixation 19 de la feuille de protection 18 sur le coussin absorbant 4.

Enfin chacun des bords longitudinaux 26 des bandes latérales 23 est fixé sur la face inférieure de la feuille de couverture 3 à proximité du premier élément élastique 16.

La feuille de protection 18 a la même longueur que la feuille de support 2 et que la feuille de couverture intérieure 3. Les bords transversaux de la feuille de protection 18 sont assemblés, notamment par collage, aux bords correspondants de la feuille support 2 et de la feuille de couverture 3.

Le mode préféré de réalisation qui vient d'être décrit cumule l'ensemble des avantages apportés par l'invention. La présence des rabats longitudinaux, constitués chacun par la portion 28 de bande latérale 23 qui s'étend à partir de la ligne de fixation 25 jusqu'au bord longitudinal 26 fixé à la face inférieure de la feuille de couverture 3 permet de créer une poche d'étanchéité disposée au-dessus du coussin absorbant 4. Cette poche forme avec la feuille de couverture 3 une double barrière continue permettant de contenir transversalement les fuites d'urine et de matières fécales.

La présence du troisième jeu d'éléments élastiques 17 sur la feuille de couverture intérieure 3 permet de former des tunnels longitudinaux 27 qui sont disposés le long des bords longitudinaux du coussin absorbant 4 et qui constituent latéralement des réservoirs tampons pour l'urine dans le cas où la présence des rabats longitudinaux ne serait pas suffisante. Ces mêmes tunnels peuvent aussi constituer des tunnels d'aération permettant un échange gazeux et donc une diminution relative de l'humidité régnant dans la poche intérieure pour autant que le matériau constitutif des rabats hydrophobes soit perméable aux gaz.

Le mode de réalisation des rabats longitudinaux avec le repli de la bande latérale 23 permet de constituer un élément supplémentaire d'étanchéité au niveau du coussin absorbant 4 puisque l'on dispose alors de trois couches successives d'un matériau hydrophobe entre le coussin absorbant 4 et la peau de l'utilisateur.

Il est à noter également que, grâce à la tension plus élevée des éléments élastiques 17 du troisième jeu par rapport à celle des éléments élastiques 16 du deuxième jeu, il s'exerce une force de rappel sur la feuille de couverture intérieure 3 telle que l'on obtient un élargissement plus important de la poche d'étanchéité dans la direction transversale de la couche-culotte 1. Cet élargissement rend plus facile la mise en place de la couche-culotte 1 sur l'utilisateur.

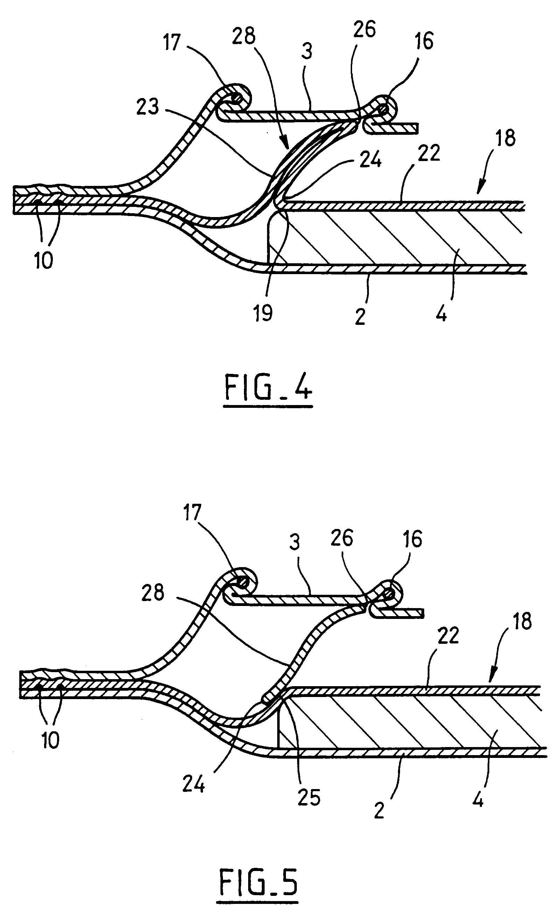

La figure 4 illustre une première variante de réalisation de l'invention qui diffère de celle décrite précédemment en ce que chaque bande latérale 23 de la feuille de protection 18 comporte un double pli en Z longitudinal, disposé le long de chaque bord longitudinal du coussin absorbant 4, au moins dans la zone d'entrejambes 7, ces plis en Z formant le jeu de rabats 28. La ligne de pliure inférieure 24 de chacun de ces plis en Z est fixée sur le bord longitudinal correspondant du coussin absorbant 4 le long d'une ligne de fixation 19 et les deux flancs de chaque pli sont fixés entre eux au niveau de cette ligne 19 de fixation et sur toute la longueur de celle-ci. Par ailleurs, la ligne de pliure supérieure 26 de chacun de ces plis en Z est fixée à la feuille de couverture intérieure 3 à proximité de l'élément élastique 16.

En outre, dans cette réalisation, la feuille de protection 18 s'étend au-delà de chacune des lignes de pliure inférieures 24 de façon à recouvrir la feuille extérieure de support 2 à laquelle elle est fixée sur leur pourtour commun. Egalement la feuille de couverture 3 recouvre, au moins partiellement, la feuille de protection 18 au-delà de chacune des lignes de pliure inférieures 24 et est fixée à la feuille de protection 18 sur leur pourtour commun.

Ceci permet d'utiliser une feuille de couverture intérieure 3 de largeur réduite égale à la largeur de la feuille de support 2 dans la zone d'entrejambes, et ainsi de faire des économies de matière. On obtient ainsi des rabats hydrophobes 28 ayant une double épaisseur, et qui de ce fait forment des barrières particulièrement efficaces contre les fuites transversales d'urine.

La feuille de protection 18 peut être composée d'un matériau non-tissé ayant une zone centrale hydrophile 22 recouvrant le coussin absorbant 4 et de zones latérales traitées, hydrophobes, pour former les rabats 28.

Dans une seconde variante de réalisation illustrée par la figure 5, la feuille de protection 18 peut être de mêmes dimensions et de même forme générale sensiblement rectangulaire que la feuille de support extérieure 2. Ainsi, la feuille de protection 18 s'étend entre la feuille de support extérieure 2 et la feuille de couverture intérieure 3 et est fixée à celles-ci sur leurs pourtours, par exemple au niveau des élastiques d'entrejambes 10.

Le matériau utilisé pour cette feuille de protection 18 peut être un non-tissé hydrophile, hydrophobe, ou hydrophile dans la zone centrale recouvrant le coussin absorbant 4 et hydrophobe dans les zones latérales adjacentes.

Les rabats hydrophobes 28 sont alors formés par des bandes séparées sensiblement rectangulaires faites par exemple d'un matériau non-tissé, d'un non-tissé enduit d'une composition thermoplastique ou d'un film thermoplastique mince afin d'être imperméable aux liquides. Les bords longitudinaux 24, 26 de ces bandes sont fixés, respectivement, d'une part pour le bord 24, à la feuille de protection 18 le long des bords longitudinaux du coussin absorbant 4 suivant une zone de collage 25 et, d'autre part pour le bord 26, à la feuille de couverture 3 à proximité des éléments élastiques 16.

L'invention n'est pas limitée aux modes de réalisation qui viennent d'être décrits à titre d'exemples non exhaustifs. En particulier la feuille de couverture 3 pourrait éventuellement être fixée à la bande latérale 23, par exemple selon deux lignes de fixation qui seraient au niveau et sur toute la longueur de la ligne 25 de fixation entre les deux flancs 23a et 23b. Sur la feuille de couverture 3 ces lignes de fixation seraient situées, symétriquement par rapport à l'axe XX, entre le premier 10 et le troisième 17 jeux de moyens élastiques. On aurait alors de part et d'autre du coussin absorbant 4 deux tunnels latéraux superposés, de section sensiblement triangulaire, ayant comme sommet commun la ligne de fixation entre la feuille de couverture 3 et la bande latérale 23.

Il est également possible de rapprocher sensiblement de la ligne de fixation la zone de recouvrement de la feuille de couverture 3 et de la feuille support 2 et donc aussi les éléments élastiques 10 du premier jeu.

## Revendications

1. Article d'hygiène absorbant jetable, notamment une couche-culotte, qui comporte un coussin absorbant (4) disposé entre une feuille extérieure support (2) imperméable aux liquides et une feuille de couverture intérieure (3) hydrophobe, les deux dites feuilles (2, 3) ayant la même forme et délimitant dans le sens longitudinal une partie centrale qui correspond à la zone d'entrejambes (7) et deux parties extrêmes, l'une avant (8) et l'autre arrière (9), qui correspondent aux zones de positionnement et de fixation autour de la taille de l'usager, la feuille support (2) et la feuille de couverture (3) étant solidarisées au moins le long de leur pourtour et la feuille de couverture (3) comportant une ouverture centrale (11) qui s'étend au-dessus du coussin absorbant (4), l'article d'hygiène comportant également des jeux de moyens élastiques, disposés symétriquement par rapport à son axe longitudinal XX, le premier jeu (10) dit jeu d'entrejambes étant fixé à la feuille support (2) et à la feuille de couverture (3) le long de leurs bords extérieurs au moins dans la zone d'entrejambes (7) et le deuxième jeu (16) étant fixé à la feuille de couverture (3) le long des bords longitudinaux de l'ouverture centrale (11), caractérisé en ce qu'il comporte un jeu de deux rabats (28) hydrophobes, symétriques par rapport à l'axe longitudinal XX, chaque rabat (28) étant constitué d'une bande d'un matériau hydrophobe et éventuellement imperméable aux liquides, dont les deux bords longitudinaux sont fixés pour l'un à proximité du bord longitudinal extérieur du coussin absorbant (4) au moins dans la zone d'entrejambes (7) et pour l'autre (26) sur la face inférieure de la feuille de couverture (3) à proximité des moyens élastiques (16) du second jeu.

2. Article d'hygiène selon la revendication 1, caractérisé en ce que le coussin absorbant étant recouvert d'une feuille de protection (18) perméable aux liquides, ladite feuille de protection (18) a une largeur qui est supérieure à celle du coussin absorbant (4) en sorte de délimiter une bande centrale (22), perméable aux liquides, et deux bandes latérales (23) hydrophobes et éventuellement imperméables aux liquides et en ce que ladite bande centrale (22) est fixée longitudinalement à proximité des deux bords extérieurs longitudinaux du coussin absorbant (4), les deux bandes latérales (23) constituant le jeu de deux rabats hydrophobes.

3. Article d'hygiène selon la revendication 2, caractérisé en ce que chaque bande latérale (23) de la feuille de protection (18) comporte un pli longitudinal, la pliure (24) correspondante étant fixée longitudinalement sur la feuille support imperméable (2) le long du coussin absorbant (4) extérieurement à celui-ci, au moins dans la zone d'entrejambes (7) et en ce que les deux flancs (23a, 23b) du pli longitudinal sont fixés l'un à l'autre au niveau et sur toute la longueur de la ligne de fixation (19) de la feuille de protection (18) sur le coussin absorbant (4).

4. Article d'hygiène selon la revendication 2, caractérisé en ce que chaque bande latérale (23) de la feuille de protection (18) comporte un double pli en Z longitudinal dont la ligne de pliure inférieure (24) est fixée sur le bord longitudinal correspondant du coussin absorbant (4), au moins dans la zone d'entrejambes, suivant une ligne de fixation (19) et en ce que les deux flancs de chaque pli sont fixés entre eux au niveau de cette ligne de fixation et sur toute la longueur de celle-ci.

5. Article d'hygiène selon l'une des revendications 1 à 4, caractérisé en ce qu'un troisième jeu de moyens élastiques (17) est fixé longitudinalement à la feuille de couverture intérieure (3) entre la ligne de fixation du rabat (28) à ladite feuille de couverture (3) et le premier jeu de moyens élastiques (10).

6. Article d'hygiène selon la revendication 5, caractérisé en ce que les moyens élastiques (17) du troisième jeu sont sensiblement à égale distance entre les moyens élastiques (10) et (16) du premier et deuxième jeux.

7. Article d'hygiène selon l'une des revendications 5 ou 6, caractérisé en ce que la force de tension des moyens élastiques (17) du troisième jeu est supérieure à celle des moyens élastiques (16) du deuxième jeu.

8. Article selon l'une des revendications 5, 6 ou 7, caractérisé en ce que les moyens élastiques du deuxième (16) et éventuellement du troisième jeux (17) sont fixés par collage, chacun dans un pli longitudinal (14, 15), notamment en forme de Z, pratiqué dans la feuille de couverture (3).

## Claims

1. Disposable absorbent sanitary article, notably a diaper, which comprises an absorbent pad (4) placed between an external backing sheet (2) which is impermeable to liquids and a hydrophobic internal covering sheet (3), the two sheets (2, 3) of identical shape and delimiting in the longitudinal direction a central part which corresponds to the crotch zone (7) and two end parts, one at the front (8) and the other at the back (9), which correspond to the zones by which the article is positioned and fixed around the wearer's waist, the backing sheet (2) and the covering sheet (3) being joined together at least along their periphery and the covering sheet (3) comprising a central opening (11) which extends above the absorbent pad (4), the sanitary article further comprising sets of elastic means, disposed symmetrically with respect to its longitudinal axis XX, the first set (10), called crotch set being fixed to the backing sheet (2) and to the covering sheet (3) along their external edges at least in the crotch zone (7) and the second set (16) being fixed to the covering sheet (3) along the longitudinal edges of the central opening (11), characterized in that it comprises a set of two hydrophobic flaps (28), which are symmetrical with respect to the longitudinal axis XX, each flap (28) being constituted of a strip of hydrophobic material, optionally impervious to liquids, of which the two longitudinal edges are fixed, for one of them close to the external longitudinal edge of the absorbent pad (4) at least in the crotch zone (7) and for the other (26) on the lower face of the covering sheet (3), close to the elastic means (16) of the second set.

2. Sanitary article according to claim 1, characterized in that, the absorbent pad being covered by a liquid-permeable protection sheet (18), said protection sheet (18) has a width which is greater than that of the absorbent pad (4) so as to delimit a liquid-permeable central strip (22), and two lateral strips (23) which are hydrophobic and optionally impervious to liquids, and in that said central strip (22) is fixed longitudinally close to the two longitudinal external edges of the absorbent pads (4), the two lateral strips (23) constituting the set of hydrophobic flaps.

3. Sanitary article according to claim 2, characterized in that each lateral strip (23) of the protection sheet (18) comprises a longitudinal fold, the corresponding folding line (24) being fixed longitudinally on the impervious backing sheet (2) along the absorbent pad (4) and externally thereto, at least in the crotch zone (7) and in that the two sides (23a, 23b) of the longitudinal fold are fixed together at the level and throughout the length of the fixing line (19) of the protection sheet (18) on the absorbent pad (4).

4. Sanitary article according to claim 2, characterized in that each side strip (23) of protection sheet (18) comprises a double longitudinal fold having a Z-shape, the lower folding line (24) of which is fixed to the corresponding longitudinal edge of the absorbent pad (4), at least in the crotch zone, along a fixing line (19) and in that the two sides of each flap are fixed together at the level and throughout the length of the fixing line.

5. Sanitary article according to one of claims 1 to 4, characterized in that a third set of elastic means (17) is fixed longitudinally to the internal covering sheet (3) between the fixing line of the flap (28) to said covering sheet (3) and the first set of elastic means (10).

6. Sanitary article according to claim 5, characterized in that the elastic means (17) of the third set are substantially at equal distance between the elastic means (10 and 16) of the first and of the second set.

7. Sanitary article according to one of claims 5 or 6, characterized in that the stretching force of the elastic means (17) of the third set is greater than that of the elastic means of the second set.

8. Article according to one of claims 5, 6 or 7, characterized in that the elastic means of the second (16) and optionally of the third set (17) are fixed by bonding, each one in a longitudinal fold (14, 15), notably Z-shaped, formed in the covering sheet (3).

## Patentansprüche

1. Hygieneartikel zum Aufnehmen von Auswurf, insbesondere eine Hosenwindel, die ein Saugkissen (4) enthält, angeordnet zwischen einer äußeren Haltefolie (2), die für Flüssigkeiten undurchlässig ist, und einer inneren, wasserabweisenden Abdeckfolie (3), derart, daß beide Folien (2, 3) dieselbe Form aufweisen und in Längsrichtung eine einem Zwischenbeinbereich (7) entsprechenden Mittenabschnitt abgrenzen sowie zwei Randabschnitte, von denen einer vorne (8) und einer hinten (9) liegt, die einem Positionier- und einem Fixierbereich um die Taille des Anwenders entsprechen, und ferner die Haltefolie (2) und die Abdeckfolie (3) als ein Stück mindestens entlang der Länge ihres äußeren Umfangs ausgebildet sind und die Abdeckfolie (3) eine Mittenöffnung aufweist, die sich oberhalb des Abdeckkissens (4) erstreckt, und der Hygieneartikel ebenso zwei Gruppen elastischer Mittel aufweist, die symmetrisch im Hinblick auf seine Längsachse XX angeordnet sind, derart, daß die erste Gruppe (10) des Zwischenbeinbereichs an der Haltefolie (2) und an der Abdeckfolie (3) entlang deren Außenränder zumindest im Zwischenbeinbereich (7) fixiert ist und die zweite Gruppe (16) an der Abdeckfolie (3) entlang den Längsrändern der Mittenöffnung (11) fixiert ist, **dadurch gekennzeichnet, daß** er eine Gruppe von zwei wasserabweisenden Schutzumschlagklappen (28) aufweist, die symmetrisch im Hinblick auf die Längsachse XX sind, derart, daß jede Schutzumschlagklappe (28) aus einem Band aus wasserabweisenden Material gebildet und gegebenenfalls undurchlässig gegenüber Flüssigkeiten ist, und daß die beiden Längsränder einerseits in der Nähe des äußeren Längsrands des Saugkissens (4) zumindest im Zwischenbeinbereich (7) und andererseits (26) an der Innenfläche der Abdeckfolie (3) in der Nähe der elastischen Mittel (16) der zweiten Gruppe fixiert sind.

2. Hygieneartikel nach Anspruch 1, **dadurch gekennzeichnet, daß** das Saugkissen von einer für Flüssigkeiten durchlässigen Schutzschicht (18) bedeckt ist, derart, daß die Schutzschicht (18) eine Größe aufweist, die über derjenigen des Saugkissens (4) liegt, so daß ein Mittenband (22) begrenzt ist, das für Flüssigkeiten durchlässig ist, sowie zwei wasserabweisende seitliche Bänder (23), die gegebenenfalls gegenüber Flüssigkeiten undurchlässig sind, derart, daß das Mittenband (22) längsseitig am Rand der beiden äußeren Längsränder des Saugkissens (4) fixiert ist und die beiden seitlichen Bänder (23) die Gruppe der beiden wasserabweisenden Schutzumschlagklappen bilden.

3. Hygieneartikel nach Anspruch 2, **dadurch gekennzeichnet, daß** jedes seitliche Band (23) der Schutzfolie (18) eine Längsfalte aufweist und der zugeordnete Falz in Längsrichtung an der undurchlässigen Haltefolie (2) entlang dem Saugkissen (4) außerhalb von diesem fixiert ist, zumindest in dem Zwischenbeinbereich (7) und daß die beiden Seitenflächen (23a, 23b) der Längsfalte wechselseitig zueinander in gleicher Höhe und über die gesamte Länge der Fixierungslinie (19) der Schutzfolie (18) an dem Saugkissen (4) fixiert sind.

4. Hygieneartikel nach Anspruch 2, **dadurch gekennzeichnet, daß** jedes seitliche Band (23) der Schutzfolie (18) eine Doppelfalte in Z-Form und in Längsrichtung aufweist, von der die tiefer liegende Faltlinie (24) am entsprechenden Längsrand des Saugkissens (4) fixiert ist, zumindest in dem Zwischenbeinbereich, und entlang einer Fixierlinie (19), und daß die beiden Seitenflächen jeder Falte aneinander auf dem Niveau dieser Fixierlinie und entlang der gesamten Länge von dieser fixiert sind.

5. Hygieneartikel nach einem der Ansprüche 1 bis 4, **dadurch gekennzeichnet, daß** eine dritte Gruppe elastischer Mittel (17) in Längsrichtung an der inneren Abdeckfolie (3) fixiert ist, und zwar zwischen der Fixierlinie der Seitenumschlagklappe (28) an der Abdeckfolie (3) und der ersten Gruppe elastischer Mittel (10).

6. Hygieneartikel nach Anspruch 5, **dadurch gekennzeichnet, daß** die elastischen Mittel (17) der dritten Gruppe mit im wesentlichen gleicher Distanz zwischen den elastischen Mitteln (10) und (16) der ersten und zweiten Gruppe vorliegen.

7. Hygieneartikel nach einem der Ansprüche 5 oder 6, **dadurch gekennzeichnet, daß** die Spannkraft der elastischen Mittel (17) der dritten Gruppe größer ist als diejenigen der elastischen Mittel (16) der zweiten Gruppe.

8. Artikel nach einem der Ansprüche 5, 6 oder 7, **dadurch gekennzeichnet, daß** die elastischen Mittel der zweiten (16) und gegebenenfalls der dritten Gruppe (17) durch Kleben fixiert sind, jeweils in einer Längsfalte (14, 15), insbesondere in Form eines Z, praktischerweise an der Abdeckfolie (3).
